# EUROPÄISCHE PATENTSCHRIFT

(11) **EP 2 051 960 B1**
(45) Veröffentlichungstag und Bekanntmachung des Hinweises auf die Patenterteilung: **08.07.2015**
(21) Anmeldenummer: 07788104.3
(22) Anmeldetag: 01.08.2007
(51) Int. Cl.: C07C 253/10, C07C 255/07

(54) **VERFAHREN ZUR HERSTELLUNG VON 3-PENTENNITRIL DURCH HYDROCYANIERUNG VON 1,3-BUTADIEN**
METHOD FOR PRODUCING 3-PENTENENITRILE BY MEANS OF THE HYDROCYANATION OF 1,3-BUTADIENE
PROCEDE DE PREPARATION DE 3-PENTENENITRILE PAR HYDROCYANURATION DE 1,3-BUTADIENE

(30) Priorität: 08.08.2006 EP 06118612
(43) Veröffentlichungstag der Anmeldung: 29.04.2009
(73) Patentinhaber: BASF SE, 67056 Ludwigshafen (DE)
(72) Erfinder: HADERLEIN, Gerd, 67269 Grünstadt (DE); ÄCHTNER, Tobias, 1012 Lausanne (CH); LEITNER, Andreas, 67071 Ludwigshafen (DE); LUYKEN, Hermann, 67069 Ludwigshafen (DE); PFAB, Peter, 67433 Neustadt (DE); SCHEIDEL, Jens, 69493 Hirschberg (DE); HAUNERT, Andrea, 68163 Mannheim (DE); GENGER, Thomas, 67245 Lambsheim (DE)
(86) Internationale Anmeldenummer: PCT/EP2007/057926
(87) Internationale Veröffentlichungsnummer: WO 2008/017626

(56) Entgegenhaltungen:
- WO-A-2005/073175

## Beschreibung

Die vorliegende Erfindung betrifft ein verbessertes Verfahren zur Herstellung von 3-Pentennitril durch Hydrocyanierung von 1,3-Butadien mit Cyanwasserstoff in Gegenwart von Nickel(0)- Komplexen mit phosphorhaltigen Liganden. Dabei wird 1,3-Butadien eingesetzt, das einen Stabilisator enthält und durch Azeotropdestillation getrocknet wird.

Adipodinitril ist ein wichtiges Ausgangsprodukt in der Nylonherstellung, das durch zweifache Hydrocyanierung von 1,3-Butadien erhalten wird. Dabei wird in einer ersten Hydrocyanierung 1,3-Butadien zu 3-Pentennitril hydrocyaniert, wobei als Nebenprodukte hauptsächlich 2-Methyl-3-butennitril, 4-Pentennitril, 2-Pentennitrile, 2-Methyl-2-butennitrile, C₉-Nitrile und Methylglutarodinitril erhalten werden. In einer zweiten, sich anschließenden Hydrocyanierung wird 3-Pentennitril mit Cyanwasserstoff zu Adipodinitril umgesetzt. Beide Hydrocyanierungen werden durch Nickel(0)-Phosphor-Komplexe katalysiert.

Eine allgemeine Übersicht über die Nickel-katalysierte Olefinhydrocyanierung ist in Tolman et al., Adv. Cat. 33, 1 - 46 (1985) beschrieben.

Die Hydrocyanierung von 1,3-Butadien unter Verwendung eines Nickelkatalysators der Formel Ni[P(OR)₃]₄ wird in US 3,496,215 beschrieben. Nachteilig an diesem Verfahren ist, dass keine geeignete Technik zur vollständigen Rückgewinnung des 1,3-Butadiens oder des Katalysators angegeben ist.

Die Durchführung der Hydrocyanierung in einem oder mehreren Reaktoren und deren Verschaltung ist in US 4,810,815 beschrieben, wobei die Möglichkeit des kontinuierlichen Betriebs von Rührkesseln oder Kaskaden von Rührkesseln erwähnt wird, jedoch in Beispielen nur eine Semibatchfahrweise im Detail beschrieben ist, woraus für den Fachmann nicht direkt abgeleitet werden kann, unter welchen Bedingungen die Fahrweise in kontinuierlichen Rührkesseln zu erfolgen hat.

Ein Verfahren zur Abtrennung von organischen phosphorhaltigen Verbindungen und ihrer Metallkomplexe von organischen Nitrilen in der Hydrocyanierung von Olefinen wird in DE 10 2004 004671 beschrieben. Die Abtrennung erfolgt dabei durch Inkontaktbringen des Produktes mit einem Cycloparaffin oder paraffinartigen Kohlenwasserstoff. Dabei bildet sich ein flüssiges mehrphasiges System.

1,3-Butadien kann polymerisieren. Daher werden dem 1,3-Butadien Stabilisatoren wie z. B. tert. Butylbrenzkatechin (TBC) zugesetzt.

Die für die Hydrocyanierung von 1,3-Butadien verwendeten Nickel(0)-Komplexe sind wasserempfindlich. Wasser enthaltendes 1,3-Butadien muß daher vor der Hydrocyanierung getrocknet werden.

Aus DE-A-10 2004 04684 ist bekannt, Wasser und einen Stabilisator enthaltendes 1,3-Butadien mit Hilfe von mikroporösen Feststoffen zu trocknen. Dabei wird nicht nur das Wasser, sondern auch der Stabilisator, zumindest teilweise von den Feststoffen adsorbiert. Als mikroporöse Feststoffe sind Aluminiumoxid und Molekularsiebe geeignet.

Heterogene Trocknungsmittel besitzen durch die Adsorption von Stabilisatoren den Nachteil, dass zumindest zeitweise nicht ausreichend oder gar nicht stabilisiertes 1,3-Butadien entsteht, das sicherheitstechnischen Beschränkungen unterliegt. So kann unstabilisiertes 1,3-Butadien nur bei Temperaturen unterhalb von 15°C verwendet werden. Hinzu kommt als weiterer Nachteil, dass die mikroporösen Adsorptionsmittel regelmäßig regeneriert werden müssen.

Darüber hinaus ist es für ein integriertes Verfahren zur Herstellung von 3-Pentennitril, bei dem sowohl 1,3-Butadien, als auch der Hydrocyanierungskatalysatorstrom zurückgeführt wird, entscheidend, dass das im molaren Überschuss gegenüber Cyanwasserstoff eingesetzte 1,3-Butadien effizient zurückgeführt wird.

Aus DE-A-10 2004 04724 ist bekannt, stabilisiertes 1,3-Butadien, das mit Hilfe von mikroporösen Feststoffen getrocknet wurde, mit Blausäure in Gegenwart von Nickel(0)-Katalysatoren zu 3-Pentennitril umzusetzen. Drei Destillationskolonnen dienen zur Abtrennung und Rückführung von nicht umgesetztem 1,3-Butadien und von Nickel(0)-Katalysator.

Aufgabe der vorliegenden Erfindung ist es somit, den zuvor genannten Nachteilen abzuhelfen und ein integriertes Verfahren zur Herstellung von 3-Pentennitril durch Hydrocyanierung von 1,3-Butadien bereitzustellen, bei dem die Verfahrensausbeute bezüglich rückgeführtem 1,3-Butadien möglichst hoch ist.

Gelöst wird diese Aufgabe durch ein Verfahren zur Herstellung von 3-Pentennitril durch Hydrocyanierung von 1,3-Butadien, das durch die folgenden Verfahrensschritte gekennzeichnet ist :
(b') Destillation von Wasser, 1- und 2-Butene und einen Stabilisator enthaltendem 1.3-Butadien in einer Destillationsvorrichtung K 4 unter Erhalt eines Stroms 15 als Sumpfprodukt, der getrocknetes 1.3-Butadien, 1- und 2-Butene und den Stabilisator enthält und eines Stroms 16 als Kopfprodukt, der ein azeotropes 1,3-Butadien/Wassergemisch enthält, Kondensation des Stroms 16 in einem Kondensator W, Überführung des erhaltenen Kondensats (Strom17) in eine Phasentrennvorrichtung, Rückführung der oberen, aus 1.3-Butadien bestehenden flüssigen Phase (Strom 18) als Rücklauf auf die Kolonne K 4 und Ausschleusung der unteren flüssigen Wasserphase (Strom 19),
(a) Umsetzung von Strom 15 in einem Reaktor R1 mit Cyanwasserstoff an mindestens einem Katalysator (Strom 6d) unter Erhalt eines Stroms 1, der 3-Pentennitril, 2-Methyl-3-butennitril, den mindestens einen Katalysator, unumgesetztes 1,3-Butadien, 1- und 2-Butene und gegebenenfalls Reste von nicht umgesetztem Cyanwasserstoff enthält,
(b) Destillation des Stromes 1 in einer Destillationsvorrichtung K1 unter Erhalt eines Stromes 2 als Kopfprodukt, der den überwiegenden Teil des 1,3-Butadiens aus Strom 1 enthält, und Erhalt eines Stromes 3 als Sumpfprodukt, der 3-Pentennitril, den mindestens einen Katalysator, 2-Methyl-3-butennitril, 1- und 2-Butene und den restlichen Teil des 1,3-Butadiens aus Strom 1 enthält, der nicht in Strom 2 abgetrennt wurde,
(c) Destillation des Stromes 3 in einer Destillationsvorrichtung K2 unter Erhalt eines Stromes 5 an einem Seitenabzug der Kolonne, der 3-Pentennitril und 2-Methyl-3-butennitril, eines Stroms 6 als Sumpfprodukt, der den mindestens einen Katalysator enthält und eines Stroms 4 als Kopfprodukt,
(d) Verdichtung des Stroms 4 im Verdichter V1, Ausschleusung eines gasförmigen Teilstroms 4b, der 1- und 2-Butene enthält, Überführung des verdichteten Stroms 4a in den Kondensator W1, gemeinsame Kondensation dieses Stromes mit Strom 2 aus (b) und Überführung des Kondensats als Strom 9 teils als Rücklauf auf die Kolonne K1 (Strom 9b), teils als Rückstrom in den Reaktor R1 (Strom 9a) und
(e) Destillative Auftrennung des Stroms 5 unter Erhalt von 3-Pentennitril und 2-Methyl-3-butennitril.

In einer bevorzugten Ausführungsform wird der in Schritt c) erhaltene Strom 6 aufgeteilt in einen Rückführungsstrom 6b und einen Ausschleusungsstrom 6a. In einer besonders bevorzugten Ausführungsform wird dem Strom 6b ein Strom 6c zugesetzt, der Frisch-Katalysator und ggf. regnerierten Katalysator aus Strom 6a enthält. Der so erhaltene Strom 6d wird als Katalysatorfeed in der Verfahrensstufe a) verwendet.

Der zuvor als überwiegender Teil des 1,3-Butadiens aus Strom 1 bezeichnete Anteil, der mit Strom 2 abgetrennt wird, bezieht sich auf einen Anteil von vorzugsweise mehr als 50 %, besonders bevorzugt mehr als 60 %, insbesondere mehr als 70 % des 1,3-Butadiens, das in Strom 1 enthalten ist. Das entsprechend verbleibende 1,3-Butadien aus Strom 1 wird über Strom 3 in den Verfahrensschritt (c) übergeführt.

Der Verfahrenschritt (b') dient zur Entwässerung des außerdem 1- und 2-Butene und einen Stabilisator enthaltenden 1,3-Butadiens. Diese Entwässerung beruht darauf, dass Butadien und Wasser ein Heteroazeotrop bilden, das bei Kondensation in eine Butadien-reiche und eine Wasser-reiche Phase zerfällt. Beide Phasen enthalten jeweils nur noch kleine Restmengen der anderen Komponente.

Als Stabilisator kann z. B. tert. Butylbrenzkatechin (TBC) oder 2,6-Di-tert.-butyl-para-Kresol verwendet werden (Ullmann's Encyclopedia of Industrial Chemistry, 6th Edition, 2000 Electronic Release, Kapitel "Butadiene-Stabilization, Storage and Transportation"). In der vorliegenden Schrift wird der Stabilisator jeweils kurz mit TBC oder Stabilisator bezeichnet.

Zur Entwässerung wird das 1,3-Butadien in eine Destillationsvorrichtung K4 überführt. In dieser Destillationvorrichtung erfolgt eine Destillation unter Erhalt eines Stroms 16 als Kopfprodukt, der das Butadien/Wasser-Azeotrop und eines Stroms 15 als Sumpfprodukt, der entwässertes 1,3-Butadien, 1- und 2-Butene und den Stabilisator enthält.

Der Verfahrensschritt (b') des erfindungsgemäßen Verfahrens kann in jeder geeigneten, dem Fachmann bekannten Vorrichtung durchgeführt werden. Für die Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen oder einstufige Verdampfer, wie Fallfilmverdampfer, Dünnschichtverdampfer, Flashverdampfer, Mehrphasenwendelrohrverdampfer, Naturumlaufverdampfer oder Zwangsumlaufentspannungsverdampfer. Die Destillation kann in mehreren, wie zwei oder drei Apparaturen, vorzugsweise in einer einzigen Apparatur durchgeführt werden.

In einer bevorzugten Ausführungsform des erfindungsgemäßen Verfahrens sind in der Destillationsvorrichtung Kolonneneinbauten mit strukturierter Packung vorhanden, die vorzugsweise zwischen 2 und 60, besonders bevorzugt zwischen 3 und 40, insbesondere zwischen 4 und 20 Trennstufen erzeugen.

In einer weiteren bevorzugten Ausführungsform des erfindungsgemäßen Verfahrens wird die Kondensation am Kopf der Destillationsvorrichtung so durchgeführt, dass ein Teilstrom vom Kopfaustrag in den Kondensator zurückgespült wird.

Die Destillation kann in einer weiteren bevorzugten Ausführungsform des erfindungsgemäßen Verfahrens mit einem Direktkondensator ausgeführt werden, so dass die Kondensation in einem Kolonnenschuss durchgeführt wird, der vorzugsweise ausgestattet ist mit einer strukturierten Kolonnenpackung, einer Fangtasse unterhalb dieser Packung, einem flüssigen Abzug aus der Fangtasse, einem an den flüssigen Abzug angeschlossenen Umpumpkreislauf mit Pumpe und Wärmetauscher sowie mindestens einer Vorrichtung zur Aufgabe des umgepumpten Flüssigstroms auf die Packung oberhalb der Fangtasse.

Die Destillation wird bei Drucken von 0,001 bis 100 bar, bevorzugt 0,01 bis 10 bar, insbesondere 0,5 bis 5 bar durchgeführt.

In Verfahrensschritt (b') wird als Kopfprodukt der Kolonne K4 ein Strom 16 erhalten, der Butadien und Wasser enthält. Dieser Strom 16 besitzt die Gleichgewichtszusammensetzung von 1,3-Butadien und Wasser beim jeweiligen Destillationsdruck.

Strom 16 wird in einen Kondensator W kondensiert. Um das im Kondensat (Strom 17) enthaltene 1,3-Butadien zu stabilisieren, kann dem Kondensator Stabilisator zugeführt werden (Strom 20). Um die Mengen an Stabilisator niedrig zu halten, wird bevorzugt bei Temperaturen unterhalb von 15 °C kondensiert.

Das Kondensat aus Kondensator W wird in eine Phasentrennvorrichtung geleitet. Dort erfolgt die Trennung der flüssigen organischen von der flüssigen wässrigen Phase. Die organische Phase (Strom 18) wird als Rücklauf auf dem Kopf der Kolonne K4 geführt. Die wässrige Phase (Strom 19) wird aus dem Verfahren ausgeschleust. Noch in der wässrigen Phase enthaltene geringe Mengen 1,3-Butadien können z. B. durch Strippen oder Erwärmen ausgetrieben und in den Kondensator zurückgefahren werden.

Als Sumpfprodukt der Kolonne K4 wird ein Strom 15 erhalten, der getrocknetes 1,3-Butadien, 1- und 2-Butene und Stabilisator enthält. Dieser Strom weist vorzugsweise einen Restgehalt an Wasser von < 1000 Gew.- ppm, bevorzugt < 100 Gew.- ppm, besonders bevorzugt < 50 Gew.-% ppm, bezogen auf den Gesamtstrom, auf.

Der Verfahrenschritt (a) umfasst die Umsetzung von durch Azeotrop-Destillation getrocknetem 1,3-Butadien (Strom 15) mit wasserfreiem Cyanwasserstoff an mindestens einem Katalysator (Strom 6d) in Gegenwart mindestens eines Stabilisators. Als Katalysator werden homogen gelöste Nickel-Komplexe verwendet. Unter dem Begriff Katalysator ist ein Gemisch aus Nickel(0)-Komplex und phosphorhaltigem Ligand zu verstehen.

Bei den Ni(0)-Komplexen, die phosphorhaltige Liganden enthalten, handelt es sich bevorzugt um homogen gelöste Nickel(0)-Komplexe.

Die phosphorhaltigen Liganden der Nickel(0)-Komplexe und die freien phosphorhaltigen Liganden sind vorzugsweise ausgewählt aus mono- oder bidentaten Phosphinen, Phosphiten, Phosphiniten und Phosphoniten.

Diese phosphorhaltigen Liganden weisen vorzugsweise die Formel I auf:

P (X¹R¹) (X²R²) (X³R³) (I)

Unter Verbindung I wird im Sinne der vorliegenden Erfindung eine einzelne Verbindung oder ein Gemisch verschiedener Verbindungen der vorgenannten Formel verstanden.

Erfindungsgemäß sind X¹, X², X³ unabhängig voneinander Sauerstoff oder Einzelbindung. Falls alle der Gruppen X¹, X² und X³ für Einzelbindungen stehen, so stellt Verbindung I ein Phosphin der Formel P(R¹R²R³) mit den für R¹, R² und R³ in dieser Beschreibung genannten Bedeutungen dar.

Falls zwei der Gruppen X¹, X² und X³ für Einzelbindungen stehen und eine für Sauerstoff, so stellt Verbindung I ein Phosphinit der Formel P(OR¹)(R²)(R³) oder P(R¹)(OR²)(R³) oder P(R¹)(R²)(OR³) mit den für R¹, R² und R³ weiter unten genannten Bedeutungen dar.

Falls eine der Gruppen X¹, X² und X³ für eine Einzelbindung steht und zwei für Sauerstoff, so stellt Verbindung I ein Phosphonit der Formel P(OR¹)(OR²)(R³) oder P(R¹)(OR²)(OR³) oder P(OR¹)(R²)(OR³) mit den für R¹, R² und R³ in dieser Beschreibung genannten Bedeutungen dar.

In einer bevorzugten Ausführungsform sollten alle der Gruppen X¹, X² und X³ für Sauerstoff stehen, so dass Verbindung I vorteilhaft ein Phosphit der Formel P(OR¹)(OR²)(OR³) mit den für R¹, R² und R³ weiter unten genannten Bedeutungen darstellt.

Erfindungsgemäß stehen R¹, R², R³ unabhängig voneinander für gleiche oder unterschiedliche organische Reste. Als R¹, R² und R³ kommen unabhängig voneinander Alkylreste, vorzugsweise mit 1 bis 10 Kohlenstoffatomen, wie Methyl, Ethyl, n-Propyl, i-Propyl, n-Butyl, i-Butyl, s-Butyl, t-Butyl, Aryl-Gruppen, wie Phenyl, o-Tolyl, m-Tolyl, p-Tolyl, 1-Naphthyl, 2-Naphthyl, oder Hydrocarbyl, vorzugsweise mit 1 bis 20 Kohlenstoffatomen, wie 1,1'-Biphenol, 1,1'-Binaphthol in Betracht. Die Gruppen R¹, R² und R³ können miteinander direkt, also nicht allein über das zentrale Phosphor-Atom, verbunden sein. Vorzugsweise sind die Gruppen R¹, R² und R³ nicht miteinander direkt verbunden.

In einer bevorzugten Ausführungsform kommen als Gruppen R¹, R² und R³ Reste ausgewählt aus der Gruppe bestehend aus Phenyl, o-Tolyl, m-Tolyl und p-Tolyl in Betracht. In einer besonders bevorzugten Ausführungsform sollten dabei maximal zwei der Gruppen R¹, R² und R³ Phenyl-Gruppen sein.

In einer anderen bevorzugten Ausführungsform sollten dabei maximal zwei der Gruppen R¹, R² und R³o-Tolyl-Gruppen sein.

Als besonders bevorzugte Verbindungen I können solche der Formel I a

(o-Tolyl-O-)_{w} (m-Tolyl-O-)ₓ (p-Tolyl-O-)_{y} (Phenyl-O-)_{z} P (Ia)

eingesetzt werden, wobei w, x, y und z eine natürliche Zahl bedeuten, und folgende Bedingungen gelten: w + x + y + z = 3 und w, z ≤ 2.

Solche Verbindungen I a sind z.B. (p-Tolyl-O-)(Phenyl-O-)₂P, (m-Tolyl-O-)(Phenyl-O-)₂P, (o-Tolyl-O-) (Phenyl-O-)₂P, (p-Tolyl-O-)₂(Phenyl-O-)P, (m-Tolyl-O-)₂(Phenyl-O-)P, (o-Tolyl-O-)₂(Phenyl-O-)P, (m-Tolyl-O-)(p-Tolyl-O)(Phenyl-O-)P, (o-Tolyl-O-)(p-Tolyl-O-)(Phenyl-O-)P, (o-Tolyl-O-)(m-Tolyl-O-)(Phenyl-O-)P, (p-Tolyl-O-)₃P, (m-Tolyl-O-)(p-Tolyl-O-)₂P, (o-Tolyl-O-)(p-Tolyl-O-)₂P, (m-Tolyl-O-)₂(p-Toluyl-O-)P, (o-Tolyl-O-)₂(p-Tolyl-O-)P, (o-Tolyl-O-)(m-Tolyl-O-)(p-Tolyl-O)P, (m-Tolyl-O-)₃P, (o-Tolyl-O-)(m-Tolyl-O-)₂P (o-Tolyl-O-)₂(m-Tolyl-O-)P, oder Gemische solcher Verbindungen.

Gemische enthaltend (m-Tolyl-O-)₃P, (m -Tolyl-O-)₂(p-Tolyl-O-)P, (m-Tolyl-O-)(p-Tolyl-O-)₂P und (p-Tolyl-O-)₃P kann man beispielsweise durch Umsetzung eines Gemisches enthaltend m-Kresol und p-Kresol, insbesondere im Molverhältnis 2 : 1, wie es bei der destillativen Aufarbeitung von Erdöl anfällt, mit einem Phosphortrihalogenid, wie Phosphortrichlorid, erhalten.

In einer anderen, ebenfalls bevorzugten Ausführungsform kommen als phosphorhaltige Liganden die in der DE-A 199 53 058 näher beschriebenen Phosphite der Formel I b in Betracht:

P (O-R¹)ₓ (O-R²)_{y} (O-R³)_{z} (O-R⁴)ₚ (I b)

mit
- R¹:: aromatischer Rest mit einem C₁-C₁₈-Alkylsubstituenten in o-Stellung zu dem Sauerstoffatom, das das Phosphoratom mit dem aromatischen System verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromatischen System verbindet, anellierten aromatischen System,
- R²:: aromatischer Rest mit einem C₁-C₁₈-Alkylsubstituenten in m-Stellung zu dem Sauerstoffatom, das das Phosphoratom mit dem aromatischen System verbindet, oder mit einem aromatischen Substituenten in m-Stellung zu dem Sauerstoffatom, das das Phosphoratom mit dem aromatischen System verbindet, oder mit einem in m-Stellung zu dem Sauerstoffatom, das das Phosphoratom mit dem aromatischen System verbindet, anellierten aromatischen System, wobei der aromatische Rest in o-Stellung zu dem Sauerstoffatom, das das Phosphoratom mit dem aromatischen System verbindet, ein Wasserstoffatom trägt,
- R³:: aromatischer Rest mit einem C₁-C₁₈-Alkylsubstituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aromatische Rest in o-Stellung zu dem Sauerstoffatom, das das Phosphoratom mit dem aromatischen System verbindet, ein Wasserstoffatom trägt,
- R⁴:: aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt,
- x:: 1 oder 2,
- y, z, p:: unabhängig voneinander 0, 1 oder 2 mit der Maßgabe, dass x+y+z+p = 3.

Bevorzugte Phosphite der Formel I b sind der DE-A 199 53 058 zu entnehmen. Als Rest R¹ kommen vorteilhaft o-Tolyl-, o-Ethyl-phenyl-, o-n-Propyl-phenyl-, o-Isopropyl-phenyl-, o-n-Butyl-phenyl-, o-sek-Butyl-phenyl-, o-tert-Butyl-phenyl-, (o-Phenyl)-Phenyl- oder 1-Naphthyl- Gruppen in Betracht.

Als Rest R² sind m-Tolyl-, m-Ethyl-phenyl-, m-n-Propyl-phenyl-, m-Isopropyl-phenyl-, m-n-Butyl-phenyl-, m-sek-Butyl-phenyl-, m-tert-Butyl-phenyl-, (m-Phenyl)-Phenyl- oder 2-Naphthyl- Gruppen bevorzugt.

Als Rest R³ kommen vorteilhaft p-Tolyl-, p-Ethyl-phenyl-, p-n-Propyl-phenyl-, p-Isopropyl-phenyl-, p-n-Butyl-phenyl-, p-sek-Butyl-phenyl-, p-tert-Butyl-phenyl- oder (p-Phenyl)-Phenyl-Gruppen in Betracht.

Rest R⁴ ist bevorzugt Phenyl. Vorzugsweise ist p gleich null. Für die Indizes x, y, z und p in Verbindung Ib ergeben sich folgende Möglichkeiten:

| x | y | z | p |
|---|---|---|---|
| 1 | 0 | 0 | 2 |
| 1 | 0 | 1 | 1 |
| 1 | 1 | 0 | 1 |
| 2 | 0 | 0 | 1 |
| 1 | 0 | 2 | 0 |
| 1 | 1 | 1 | 0 |
| 1 | 2 | 0 | 0 |
| 2 | 0 | 1 | 0 |
| 2 | 1 | 0 | 0 |

Bevorzugte Phosphite der Formel I b sind solche, in denen p gleich null ist sowie R¹, R² und R³ unabhängig voneinander ausgewählt sind aus o-Isopropyl-phenyl, m-Tolyl und p-Tolyl, und R⁴ Phenyl ist.

Besonders bevorzugte Phosphite der Formel I b sind solche, in denen R¹ der o-Isopropyl-phenyl-Rest, R² der m-Tolylrest und R³ der p-Tolylrest ist mit den in der vorstehenden Tabelle genannten Indizes; außerdem solche, in denen R¹ der o-Tolylrest, R² der m-Tolylrest und R³ der p-Tolylrest ist mit den in der Tabelle genannten Indizes; weiterhin solche, in denen R¹ der 1-Naphthylrest, R² der m-Tolylrest und R³ der p-Tolylrest ist mit den in der Tabelle genannten Indizes; außerdem solche, in denen R¹ der o-Tolylrest, R² der 2-Naphthylrest und R³ der p-Tolylrest ist mit den in der Tabelle genannten Indizes; und schließlich solche, in denen R¹ der o-Isopropyl-phenyl-Rest, R² der 2-Naphthylrest und R³ der p-Tolylrest ist mit den in der Tabelle genannten Indizes; sowie Gemische dieser Phosphite.

Phosphite der Formel I b können erhalten werden, indem man
a) ein Phosphortrihalogenid mit einem Alkohol ausgewählt aus der Gruppe bestehend aus R¹OH, R²OH, R³OH und R⁴OH oder deren Gemische umsetzt unter Erhalt eines Dihalogenophosphorigsäuremonoesters,
b) den genannten Dihalogenophosphorigsäuremonoester mit einem Alkohol ausgewählt aus der Gruppe bestehend aus R¹OH, R²OH, R³OH und R⁴OH oder deren Gemische umsetzt unter Erhalt eines Monohalogenophosphorigsäurediesters und
c) den genannten Monohalogenophosphorigsäurediester mit einem Alkohol ausgewählt aus der Gruppe bestehend aus R¹OH, R²OH, R³OH und R⁴OH oder deren Gemische umsetzt unter Erhalt eines Phosphits der Formel I b.

Die Umsetzung kann in drei getrennten Schritten durchgeführt werden. Ebenso können zwei der drei Schritte kombiniert werden, also a) mit b) oder b) mit c). Alternativ können alle der Schritte a), b) und c) miteinander kombiniert werden.

Dabei kann man geeignete Parameter und Mengen der Alkohole ausgewählt aus der Gruppe bestehend aus R¹OH, R²OH, R³OH und R⁴OH oder deren Gemische durch einige einfache Vorversuche leicht ermitteln.

Als Phosphortrihalogenid kommen grundsätzlich alle Phosphortrihalogenide, vorzugsweise solche, in denen als Halogenid Cl, Br, I, insbesondere Cl, eingesetzt wird, sowie deren Gemische in Betracht. Es können auch Gemische verschiedener gleich oder unterschiedlich halogensubstituierter Phosphine als Phosphortrihalogenid eingesetzt werden. Besonders bevorzugt ist PCl₃. Weitere Einzelheiten zu den Reaktionsbedingungen bei der Herstellung der Phosphite I b und zur Aufarbeitung sind der DE-A 199 53 058 zu entnehmen.

Die Phosphite I b können auch in Form eines Gemisches verschiedener Phosphite I b als Ligand verwendet werden. Ein solches Gemisch kann beispielsweise bei der Herstellung der Phosphite I b anfallen.

Es ist allerdings bevorzugt, dass der phosphorhaltige Ligand mehrzähnig, insbesondere zweizähnig ist. Daher weist der verwendete Ligand vorzugsweise die Formel II 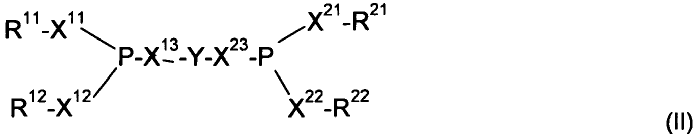 auf, worin bedeuten
- X¹¹, X¹², X¹³, X²¹, X²², X²³: unabhängig voneinander Sauerstoff oder Einzelbindung
- R¹¹, R¹²: unabhängig voneinander gleiche oder unterschiedliche, einzelne oder verbrückte organische Reste
- R²¹, R²²: unabhängig voneinander gleiche oder unterschiedliche, einzelne oder verbrückte organische Reste,
- Y: Brückengruppe

Unter Verbindung II wird im Sinne der vorliegenden Erfindung eine einzelne Verbindung oder ein Gemisch verschiedener Verbindungen der vorgenannten Formel verstanden.

In einer bevorzugten Ausführungsform können X¹¹, X¹², X¹³, X²¹, X²², X²³ Sauerstoff darstellen. In einem solchen Fall ist die Brückengruppe Y mit Phosphit-Gruppen verknüpft.

In einer anderen bevorzugten Ausführungsform können X¹¹ und X¹² Sauerstoff und X¹³ eine Einzelbindung oder X¹¹ und X¹³ Sauerstoff und X¹² eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²¹ und X²² Sauerstoff und X²³ eine Einzelbindung oder X²¹ und X²³ Sauerstoff und X²² eine Einzelbindung oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphonits, Phosphinits oder Phosphins, vorzugsweise eines Phosphonits, sein kann.

In einer anderen bevorzugten Ausführungsform können X¹³ Sauerstoff und X¹¹ und X¹² eine Einzelbindung oder X¹¹ Sauerstoff und X¹² und X¹³ eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphinits oder Phosphins, vorzugsweise eines Phosphinits, sein kann.

In einer anderen bevorzugten Ausführungsform können X¹¹, X¹² und X¹³ eine Einzelbindung darstellen, so dass das mit X¹¹, X¹² und X¹³ umgebene Phosphoratom Zentralatom eines Phosphins ist. In einem solchen Fall können X²¹, X²² und X²³ Sauerstoff oder X²¹, X²² und X²³ eine Einzelbindung darstellen, so dass das mit X²¹, X²² und X²³ umgebene Phosphoratom Zentralatom eines Phosphits oder Phosphins, vorzugsweise eines Phosphins, sein kann.

Als Brückengruppe Y kommen vorzugsweise substituierte, beispielsweise mit C₁-C₄-Alkyl, Halogen, wie Fluor, Chlor, Brom, halogeniertem Alkyl, wie Trifluormethyl, Aryl, wie Phenyl, oder unsubstituerte Arylgruppen in Betracht, vorzugsweise solche mit 6 bis 20 Kohlenstoffatomen im aromatischen System, insbesondere Pyrocatechol, Bis(phenol) oder Bis(naphthol).

Die Reste R¹¹ und R¹² können unabhängig voneinander gleiche oder unterschiedliche organische Reste darstellen. Vorteilhaft kommen als Reste R¹¹ und R¹² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R²¹ und R²² können unabhängig voneinander gleiche oder unterscheidliche organische Reste darstellen. Vorteilhaft kommen als Reste R²¹ und R²² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R¹¹ und R¹² können einzeln oder verbrückt sein. Auch die Reste R²¹ und R²² können einzeln oder verbrückt sein. Die Reste R¹¹, R¹², R²¹ und R²² können alle einzeln, zwei verbrückt und zwei einzeln oder alle vier verbrückt sein in der beschriebenen Art.

In einer besonders bevorzugten Ausführungsform kommen die in US 5,723,641 genannten Verbindungen der Formel I, II, III, IV und V in Betracht. In einer besonders bevorzugten Ausführungsform kommen die in US 5,512,696 genannten Verbindungen der Formel I, II, 111 IV, V, VI und VII, insbesondere die dort in den Beispielen 1 bis 31 eingesetzten Verbindungen, in Betracht. In einer besonders bevorzugten Ausführungsform kommen die in US 5,821,378 genannten Verbindungen der Formel I, II, III, IV, V, VI, VII, VIII, IX, X, XI, XII, XIII, XIV und XV, insbesondere die dort in den Beispielen 1 bis 73 eingesetzten Verbindungen, in Betracht.

In einer besonders bevorzugten Ausführungsform kommen die in US 5,512,695 genannten Verbindungen der Formel I, II, III, IV, V und VI, insbesondere die dort in den Beispielen 1 bis 6 eingesetzten Verbindungen, in Betracht. In einer besonders bevorzugten Ausführungsform kommen die in US 5,981,772 genannten Verbindungen der Formel I, II, III, IV, V, VI, VII, VIII, IX, X, XI, XII, XIII und XIV, insbesondere die dort in den Beispielen 1 bis 66 eingesetzten Verbindungen, in Betracht.

In einer besonders bevorzugten Ausführungsform kommen die in US 6,127,567 genannten Verbindungen und dort in den Beispielen 1 bis 29 eingesetzten Verbindungen in Betracht. In einer besonders bevorzugten Ausführungsform kommen die in US 6,020,516 genannten Verbindungen der Formel I, II, III, IV, V, VI, VII, VIII, IX und X, insbesondere die dort in den Beispielen 1 bis 33 eingesetzten Verbindungen, in Betracht. In einer besonders bevorzugten Ausführungsform kommen die in US 5,959,135 genannten Verbindungen und dort in den Beispielen 1 bis 13 eingesetzten Verbindungen in Betracht.

In einer besonders bevorzugten Ausführungsform kommen die in US 5,847,191 genannten Verbindungen der Formel I, II und III in Betracht. In einer besonders bevorzugten Ausführungsform kommen die in US 5,523,453 genannten Verbindungen, insbesondere die dort in Formel 1, 2, 3, 4, 5, 6, 7, 8, 9, 10, 11, 12, 13, 14, 15, 16, 17, 18, 19, 20 und 21 dargestellten Verbindungen, in Betracht. In einer besonders bevorzugten Ausführungsform kommen die in WO 01/14392 genannten Verbindungen, vorzugsweise die dort in Formel V, VI, VII, VIII, IX, X, XI, XII, XIII, XIV, XV, XVI, XVII, XXI, XXII, XXIII dargestellten Verbindungen, in Betracht.

In einer besonders bevorzugten Ausführungsform kommen die in WO 98/27054 genannten Verbindungen in Betracht. In einer besonders bevorzugten Ausführungsform kommen die in WO 99/13983 genannten Verbindungen in Betracht. In einer besonders bevorzugten Ausführungsform kommen die in WO 99/64155 genannten Verbindungen in Betracht.

In einer besonders bevorzugten Ausführungsform kommen die in der deutschen Patentanmeldung DE 100 380 37 genannten Verbindungen in Betracht. In einer besonders bevorzugten Ausführungsform kommen die in der deutschen Patentanmeldung DE 100 460 25 genannten Verbindungen in Betracht. In einer besonders bevorzugten Ausführungsform kommen die in der deutschen Patentanmeldung DE 101 502 85 genannten Verbindungen in Betracht.

In einer besonders bevorzugten Ausführungsform kommen die in der deutschen Patentanmeldung DE 101 502 86 genannten Verbindungen in Betracht. In einer besonders bevorzugten Ausführungsform kommen die in der deutschen Patentanmeldung DE 102 071 65 genannten Verbindungen in Betracht. In einer weiteren besonders bevorzugten Ausführungsform der vorliegenden Erfindung kommen die in der US 2003/0100442 A1 genannten phosphorhaltigen Chelatliganden in Betracht.

In einer weiteren besonders bevorzugten Ausführungsform der vorliegenden Erfindung kommen die in der nicht vorveröffentlichten deutschen Patentanmeldung Aktenzeichen DE 103 50 999 genannten phosphorhaltigen Chelatliganden in Betracht.

Die beschriebenen Verbindungen I, I a, I b und II sowie deren Herstellung sind an sich bekannt. Als phosphorhaltiger Ligand können auch Mischungen, enthaltend mindestens zwei der Verbindungen I, I a, I b und II, eingesetzt werden.

In einer besonders bevorzugten Ausführungsform des erfindungsgemäßen Verfahrens ist der phosphorhaltige Ligand des Nickel(0)-Komplexes und/oder der freie phosphorhaltige Ligand ausgewählt aus Tritolylphosphit, bidentaten phosphorhaltigen Chelatliganden, sowie den Phosphiten der Formel I b

P (O-R¹)ₓ (O-R²)_{y} (O-R³)_{z} (O-R⁴)ₚ (I b)

worin R¹, R² und R³ unabhängig voneinander ausgewählt sind aus o-Isopropyl-phenyl, m-Tolyl und p-Tolyl, R⁴ Phenyl ist; x gleich 1 oder 2 ist, und y, z, p unabhängig voneinander 0, 1 oder 2 sind mit der Maßgabe, dass x+y+z+p = 3 ist; und deren Mischungen.

Der Verfahrensschritt (a) des erfindungsgemäßen Verfahrens kann in jeder geeigneten, dem Fachmann bekannten Vorrichtung durchgeführt werden. Für die Reaktion kommen somit übliche Apparaturen in Betracht, wie sie beispielsweise in: Kirk-Othmer, Encyclopedia of Chemical Technology, 4. Ed., Vol. 20, John Wiley & Sons, New York, 1996, Seiten 1040 bis 1055 beschrieben sind, wie Rührkesselreaktoren, Schlaufenreaktoren, Gasumlaufreaktoren, Blasensäulenreaktoren oder Rohrreaktoren, jeweils gegebenenfalls mit Vorrichtungen zur Abfuhr von Reaktionswärme. Die Reaktion kann in mehreren, wie zwei oder drei, Apparaturen durchgeführt werden.

In einer bevorzugten Ausführungsform des erfindungsgemäßen Verfahrens haben sich Reaktoren mit Rückvermischungscharakteristik oder Kaskaden von Reaktoren mit Rückvermischungscharakteristik als vorteilhaft erwiesen. Als besonders vorteilhaft haben sich Schlaufenreaktoren erwiesen (s. DE 10 2004 004673).

Die Hydrocyanierung kann in Gegenwart oder in Abwesenheit von einem Lösemittel durchgeführt werden. Wenn ein Lösemittel verwendet wird, so sollte das Lösemittel bei der gegebenen Reaktionstemperatur und dem gegebenen Reaktionsdruck flüssig und inert gegenüber den ungesättigten Verbindungen und dem mindestens einen Katalysator sein. Im Allgemeinen werden als Lösemittel Kohlenwasserstoffe, beispielsweise Benzol oder Xylol, oder Nitrile, beispielsweise Acetonitril, Benzonitril oder Pentennitrile wie 2-, 3- und 4-Pentennitrile verwendet.

Die Reaktion kann in Batchfahrweise, kontinuierlich oder im Semibatchbetrieb durchgeführt werden.

Vorzugsweise wird die Hydrocyanierung kontinuierlich in einem oder mehreren gerührten Verfahrensschritten durchgeführt. Wenn eine Mehrzahl von Verfahrensschritten verwendet wird, so ist es bevorzugt, dass die Verfahrensschritte in Serie geschaltet sind. Dabei wird das Produkt von einem Verfahrensschritt direkt in den nächsten Verfahrensschritt überführt. Der Cyanwasserstoff kann direkt in den ersten Verfahrensschritt oder zwischen den einzelnen Verfahrensschritten der Hydrocyanierungs-Reaktion (a) zugeführt werden.

Die Reaktion wird vorzugsweise bei absoluten Drücken von 0,1 bis 100 MPa, besonders bevorzugt 0,5 bis 50 MPa, insbesondere 1 bis 5 MPa, durchgeführt. Die Reaktion wird vorzugsweise bei Temperaturen von 273 bis 473 K, besonders bevorzugt 313 bis 423 K, insbesondere bei 333 bis 393 K, durchgeführt. Dabei haben sich durchschnittliche mittlere Verweilzeiten der flüssigen Reaktorphase im Bereich von 0,001 bis 100 Stunden, vorzugsweise 0,05 bis 20 Stunden, besonders bevorzugt 0,1 bis 5 Stunden, jeweils pro Reaktor, als vorteilhaft erwiesen.

Die Reaktion kann in einer Ausführungsform in flüssiger Phase in Gegenwart einer Gasphase und gegebenenfalls einer festen suspendierten Phase ausgeführt werden. Dabei können die Ausgangsstoffe Cyanwasserstoff und 1,3-Butadien jeweils flüssig oder gasförmig zudosiert werden.

Die Reaktion kann in einer weiteren Ausführungsform in flüssiger Phase durchgeführt werden, wobei der Druck im Reaktor so bemessen ist, dass alle Einsatzstoffe wie 1,3-Butadien, Cyanwasserstoff und der mindestens eine Katalysator flüssig zudosiert werden und in der Reaktionsmischung in flüssiger Phase vorliegen. Dabei kann eine feste suspendierte Phase im Reaktionsgemisch vorliegen, die auch zusammen mit dem mindestens einen Katalysator zudosiert werden kann, beispielsweise bestehend aus Abbauprodukten des Katalysatorsystems, enthaltend unter anderem Nickel(II)-Verbindungen.

Im Verfahrensschritt (a) wird ein Strom 1, der 3-Pentennitril, 2-Methyl-3-butennitril, den mindestens einen Katalysator, Stabilisator und nicht umgesetztes 1,3-Butadien, 1- und 2-Butene sowie Reste von nicht umgesetztem Cyanwasserstoff enthält, erhalten. Dieser Strom 1 weist vorzugsweise die folgende Zusammensetzung auf: 1 bis 80 Gew.-%, besonders bevorzugt 5 bis 50 Gew.-%, des mindestens einen Katalysators, 0,1 bis 50 Gew.-%, besonders bevorzugt 1 bis 25 Gew.-%, 1,3-Butadien, 1 bis 80 Gew.-%, besonders bevorzugt 10 bis 50 Gew.-%, Pentennitrile, umfassend trans-3-Pentennitril, 2-Methyl-3-butennitril sowie weitere Pentennitrilisomere und 0,1 Gew.-ppm bis 10 Gew.-%, besonders bevorzugt 1 Gew.-ppm bis 1 Gew.-%, Cyanwasserstoff jeweils bezogen auf die Gesamtmasse des Stromes 1.

Der Strom 1, der 3-Pentennitril, 2-Methyl-3-butennitril, den mindestens einen Katalysator, Stabilisator und nicht umgesetztes 1,3-Butadien, 1- und 2-Butene enthält, wird anschließend in Verfahrensschritt (b) in eine Destillationsvorrichtung K1 überführt. In dieser Destillationsvorrichtung erfolgt eine Destillation des Stromes 1 unter Erhalt eines an 1,3-Butadien und 1- und 2-Butenen reichen Stromes 2 als Kopfprodukt, eines an 1,3-Butadien armen Stromes 3 als Sumpfprodukt, der 3-Pentennitril, den mindestens einen Katalysator, Stabilisator und 2-Methyl-3-butennitril enthält.

Der Verfahrensschritt (b) des erfindungsgemäßen Verfahrens kann in jeder geeigneten, dem Fachmann bekannten Vorrichtung durchgeführt werden. Für die Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen oder einstufige Verdampfer, wie Fallfilmverdampfer, Dünnschichtverdampfer, Flashverdampfer, Mehrphasenwendelrohrverdampfer, Naturumlaufverdampfer oder Zwangsumlaufentspannungsverdampfer. Die Destillation kann in mehreren, wie zwei oder drei Apparaturen, vorzugsweise in einer einzigen Apparatur durchgeführt werden.

In einer bevorzugten Ausführungsform des erfindungsgemäßen Verfahrens sind in der Destillationsvorrichtung Kolonneneinbauten mit strukturierter Packung vorhanden, die vorzugsweise zwischen 2 und 60, besonders bevorzugt zwischen 3 und 40, insbesondere zwischen 4 und 20, Trennstufen erzeugen.

In einer besonders bevorzugten Ausführungsform des erfindungsgemäßen Verfahrens wird die mindestens eine zur Destillationsvorrichtung von Verfahrensschritt (b) gehörige Verdampferstufe so ausgeführt, dass das zu verdampfende Material möglichst wenig thermische Schädigung erleidet, wie es beispielsweise durch Fallfilmverdampfer, Mehrphasenwendelrohrverdampfer, Dünnschichtverdampfer oder Kurzwegverdampfer durch kurze Kontaktzeiten des Materials an der Verdampferoberfläche und möglichst geringe Temperaturen der Verdampferoberflächen erreicht wird.

In einer bevorzugten Ausführungsform des erfindungsgemäßen Verfahrens wird die Destillationsvorrichtung von Verfahrensschritt (b) mit einem geteilten Sumpf betrieben, wobei man aus einem ersten Sumpf der betreffenden Destillationskolonne einen im Verhältnis zum Strom 3 im Allgemeinen um ein Vielfaches größeren Umlaufstrom zum Verdampfer fährt, den flüssigen Ablaufstrom aus dem Verdampfer jedoch nicht direkt in den ersten Sumpf zurückgibt, sondern in einen zweiten Sumpf, der vom ersten Sumpf getrennt ist, auffängt, aus dem zweiten Sumpf den Strom 3 erhält und den verbleibenden Überschuss vom Verdampferumlaufstrom in den ersten Sumpf überlaufen lässt, wobei als Strom 3 aus dem zweiten Sumpf eine Mischung erhalten wird, die gegenüber dem aus dem ersten Sumpf abgezogenen Verdampferumlaufstrom an Leichtsiedern abgereichert ist. Als Verdampfer wird dabei vorzugsweise ein Fallfilmverdampfer verwendet.

In einer weiteren bevorzugten Ausführungsform des erfindungsgemäßen Verfahrens wird die Destillation bei mittleren Verweilzeiten der flüssigen Phase im Sumpfbereich der ein oder mehreren Destillationsapparaturen in dem Verfahrensschritt (b) von zusammen weniger als 10 Stunden, besonders bevorzugt weniger als 5 Stunden, insbesondere weniger als 1 Stunde, durchgeführt.

In einer weiteren bevorzugten Ausführungsform des erfindungsgemäßen Verfahrens wird die Kondensation am Kopf der Destillationsvorrichtung so durchgeführt, dass ein Teilstrom vom Kopfaustrag in den Kondensator zurückgespült wird.

Die Destillation kann in einer weiteren bevorzugten Ausführungsform des erfindungsgemäßen Verfahrens mit einem Direktkondensator ausgeführt werden, so dass die Kondensation in einem Kolonnenschuss durchgeführt wird, der vorzugsweise ausgestattet ist mit einer strukturierten Kolonnenpackung, einer Fangtasse unterhalb dieser Packung, einem flüssigen Abzug aus der Fangtasse, einem an den flüssigen Abzug angeschlossenen Umpumpkreislauf mit Pumpe und Wärmetauscher sowie mindestens einer Vorrichtung zur Aufgabe des umgepumpten Flüssigstroms auf die Packung oberhalb der Fangtasse.

Die in Verfahrensschritt (b) verwendete Destillationsvorrichtung K1 umfasst eine Destillationskolonne mit Abtriebsteil, wobei die Destillationskolonne vorzugsweise 2 bis 60, besonders bevorzugt 3 bis 40, insbesondere 4 bis 20, theoretische Trennstufen aufweist.

Um eine möglichst hohe Verfahrensausbeute bezüglich 1,3-Butadien trotz der nur teilweise erfolgten Umsetzung in Schritt (a) zu erreichen, ist es bevorzugt, dass der an 1,3-Butadien reiche Strom 2 in den Verfahrensschritt (a) zurückgeführt wird. Diese Rückführung erfolgt jedoch nicht direkt in den Reaktor R₁. Außerdem wird nicht der gesamte Strom 2 in den Verfahrensschritt (a) zurückgeführt.

Strom 2 wird zunächst in Verfahrensschritt (d) in einem Kondensator W1 bevorzugt gemeinsam mit Strom 4a aus Schritt d) kondensiert. Aus dem Kondensator wird ein flüssiger Strom 9 ausgeschleust, der ganz überwiegend 1,3-Butadien enthält und zum Teil als Rücklauf auf die Kolonne K1 (Strom 9b), zum Teil in dem Reaktor R1 (Strom 9a) geführt wird 1- und 1-Butene pegeln sich im Kreislauf des 1,3-Butadiens des erfindungsgemäßen Verfahrens auf, je nachdem, wie gut die Effizienz der Rückführung ist. Je vollständiger 1,3-Butadien zurückgeführt wird, desto eher machen sich die Aufpegelungen bemerkbar.

Der Strom 2 wird somit vorzugsweise so erzeugt, dass er weniger als 50 Gew.-%, besonders bevorzugt weniger als 25 Gew.-%, insbesondere weniger als 15 Gew.-%, und vorzugsweise mehr als 1 Gew.-%, besonders bevorzugt mehr als 2,5 Gew.-%, insbesondere mehr als 5 Gew.-%, in Summe trans-2-Buten, cis-2-Buten und 1-Buten enthält. Der Rest ist im wesentlichen 1,3-Butadien.

Eine Möglichkeit, die Aufpegelung der Buten-Isomere auf den gewünschten Wert zu begrenzen, ist aus dem Kondensator W1 einen gasförmigen Teilstrom als Strom 13 auszuschleusen. Dies ist gegebenenfalls mit Verlusten an 1,3-Butadien verbunden, da einerseits der cis-2-Buten-Gehalt im Kreislaufstrom 2 nicht zu hoch ansteigen darf, andererseits bei dieser Ausschleusung immer 1,3-Butadien zwangsweise ausgeschleust wird. Der Strom 13 wird vorzugsweise gasförmig entnommen.

Eine weitere Möglichkeit zur Abtrennung von Buten-Isomeren aus dem Butadienkreislauf besteht erfindungsgemäß darin, die Destillationsvorrichtung K1 so zu betreiben, dass unterhalb des Zulaufs von Strom 1 Trennstufen wirksam sind, die eine Anreicherung von cis-2-Buten gegenüber 1,3-Butadien in Strom 3 zulassen. Anstelle einer Ausschleusung aus Strom 2 erfolgt dann eine Ausschleusung in Verfahrenschritt (d) in Form des Stromes 4b, der wie nachfolgend in einer bevorzugten Ausführungsform beschrieben aus Strom 3 erzeugt wird

Die Ausschleusungen erfolgen vorzugsweise gasförmig.

Der absolute Druck in Verfahrensschritt (b) beträgt vorzugsweise 0,001 bis 100 bar, besonders bevorzugt 0,01 bis 10 bar, insbesondere 0,5 bis 5 bar. Die Destillation wird so durchgeführt, dass die Temperatur im Sumpf der Destillationsvorrichtung vorzugsweise 30 bis 140 °C, besonders bevorzugt 50 bis 130 °C, insbesondere 60 bis 120 °C, beträgt. Die Destillation wird so durchgeführt, dass die Kondensationstemperatur am Kopf der Destillationsvorrichtung vorzugsweise -50 bis 140 °C, besonders bevorzugt -15 bis 60 °C, insbesondere 5 bis 45 °C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten.

Das Rücklaufverhältnis am Kopf der Destillationsvorrichtung wird vorzugsweise so eingestellt, dass der Strom 2 1 bis 1000 ppm, besonders bevorzugt 5 bis 500 ppm, insbesondere 10 bis 200 ppm, 2 Methyl-3-butennitril enthält.

In Verfahrensschritt (b) wird ein an 1,3-Butadien reicher Strom 2 als Kopfprodukt und ein an 1,3-Butadien armer Strom 3 als Sumpfprodukt erhalten. Die Bezeichnung der Ströme als an 1,3-Butadien reich bzw. arm bezieht sich dabei auf den Gehalt an 1,3-Butadien des in Verfahrensschritt (b) eingesetzten Stromes 1.

In einer bevorzugten Ausführungsform des erfindungsgemäßen Verfahrens enthält der an 1,3-Butadien reiche Strom 2 in Summe 50 bis 100 Gew.-%, besonders bevorzugt 80 bis 100 Gew.-%, insbesondere 85 bis 99 Gew.-%, 1,3-Butadien und Buten-Isomere sowie in Summe 0 bis 50 Gew.-%, besonders bevorzugt 0 bis 20 Gew.-%, insbesondere 10 Gew.-ppm bis 1 Gew.-%, Pentennitril-Isomere, von denen im wesentlichen 2-Methyl-3-butennitril und trans-3-Pentennitril im Strom 2 vertreten sind.

In einer bevorzugten Ausführungsform des erfindungsgemäßen Verfahrens enthält der an 1,3-Butadien arme Strom 3 in Summe 0 bis 50 Gew.-%, besonders bevorzugt 1 bis 30 Gew.-%, insbesondere 2 bis 20 Gew.-%, 1,3-Butadien und Buten-Isomere und 1 Gew. ppm bis 10 Gew.-%, besonders bevorzugt 10 Gew. ppm bis 5 Gew.-%, insbesondere 100 Gew. ppm bis 2 Gew.-% Stabilisator, bezogen auf die Gesamtmasse des Stromes 3. In einer besonders bevorzugten Ausführungsform des erfindungsgemäßen Verfahrens werden die zuvor genannten Spezifikationen an 1,3-Butadien sowohl im Strom 2 als auch im Strom 3 erreicht.

Der aus Verfahrensschritt (b) stammende, an 1,3-Butadien arme Strom 3, der 3-Pentennitril, den mindestens einen Katalysator und mindestens einen Stabilisator und 2-Methyl-3-butennitril enthält, wird anschließend in Verfahrensschritt (c) in eine Destillationsvorrichtung K 2 überführt. In dieser Destillationsvorrichtung erfolgt eine Destillation des Stromes 3 unter Erhalt eines Stromes 4 als Kopfprodukt, der 1,3-Butadien enthält, eines Stromes 5 an einem Seitenabzug der Kolonne, der 3-Pentennitril und 2-Methyl-3-butennitril enthält, und eines Stromes 6 als Sumpfprodukt, der den mindestens einen Katalysator und mindestens einen Stabilisator enthält.

Der Verfahrensschritt (c) des erfindungsgemäßen Verfahrens kann in jeder geeigneten, dem Fachmann bekannten Vorrichtung durchgeführt werden. Für diese Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334 - 348 beschrieben sind, wie Siebbodenkolonnen, Glockenbodenkolonnen, Packungskolonnen, Füllkörperkolonnen oder einstufige Verdampfer, wie Fallfilmverdampfer, Dünnschichtverdampfer, Flashverdampfer, Mehrphasenwendelrohrverdampfer, Naturumlaufverdampfer oder Zwangsumlaufentspannungsverdampfer. Die Destillation kann in mehreren, wie zwei oder drei Apparaturen, vorzugsweise in einer Apparatur durchgeführt werden.

In einer besonders bevorzugten Ausführungsform wird als Destillationsvorrichtung in Verfahrensschritt (c) mindestens eine Destillationskolonne ausgewählt, die einen Abtriebsteil umfasst, besonders bevorzugt nur eine Destillationskolonne, die nur einen Abtriebsteil aufweist.

Die Destillationsvorrichtung ist vorzugsweise mit einer strukturierten Packung ausgestattet, die 2 bis 50, besonders bevorzugt 3 bis 40, insbesondere 4 bis 30, theoretische Trennstufen erzeugt.

In einer besonders bevorzugten Ausführungsform des erfindungsgemäßen Verfahrens werden die mindestens eine zur Destillationsvorrichtung von Verfahrensschritt (c) gehörigen Verdampferstufen so ausgeführt, dass das zu verdampfende Material möglichst wenig thermische Schädigung erleidet, wie es beispielsweise durch Fallfilmverdampfer, Mehrphasenwendelrohrverdampfer, Dünnschichtverdampfer oder Kurzwegverdampfer, durch kurze Kontaktzeiten des Materials an der Verdampferoberfläche und möglichst geringe Temperaturen der Verdampferoberflächen erreicht wird.

In einer weiteren bevorzugten Ausführungsform des erfindungsgemäßen Verfahrens wird die Destillation bei mittleren Verweilzeiten der flüssigen Phase im Sumpfbereich der Destillationsapparaturen in dem Verfahrensschritt (c) von zusammen weniger als 10 Stunden, besonders bevorzugt weniger als 5 Stunden, insbesondere weniger als 1 Stunde, durchgeführt.

In einer besonders bevorzugten Ausführungsform des erfindungsgemäßen Verfahrens wird die Destillation bei mittleren Verweilzeiten der flüssigen Phase im Sumpfbereich der Destillationsapparaturen in den Verfahrensschritten (b) und (c) von zusammen weniger als 10 Stunden, besonders bevorzugt weniger als 5 Stunden, insbesondere weniger als 1 Stunde, durchgeführt.

Der absolute Druck in Verfahrensschritt (c) beträgt vorzugsweise 0,001 bis 10 bar, besonders bevorzugt 0,010 bis 1 bar, insbesondere 0,020 bis 0,5 bar. Die Destillation wird so durchgeführt, dass die Temperatur im Sumpf der Destillationsvorrichtung vorzugsweise 30 bis 140 °C, besonders bevorzugt 40 bis 130°C, insbesondere 50 bis 120°C, beträgt. Die Destillation wird so durchgeführt, dass die Kondensationstemperatur am Kopf der Destillationsvorrichtung vorzugsweise -20 bis 140 °C, besonders bevorzugt -10 bis 80°C, insbesondere -5 bis 60 °C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten.

In der Destillation des Verfahrensschrittes (c) wird ein Strom 4 als Kopfprodukt erhalten. Dieser Strom 4 enthält vorzugsweise in Summe 50 bis 100 Gew.-%, besonders bevorzugt 80 bis 100 Gew.-%, insbesondere 90 bis 99,9 Gew.-%, 1,3-Butadien und Buten-Isomere sowie in Summe 0 bis 50 Gew.-%, besonders bevorzugt 0 bis 20 Gew.-%, insbesondere 10 Gew.-ppm bis 10 Gew.-%, Pentennitril-Isomere, von denen im Wesentlichen 2-Methyl-3-butennitril und trans-3-Pentennitril im Strom 4 vertreten sind.

Der Gehalt an trans-2-Buten, cis-2-Buten und 1-Buten in Summe in Strom 4 bzw. 4a beträgt vorzugsweise mehr als 2 Gew.-%, besonderns bevorzugt mehr als 10 Gew.-%, insbesondere mehr als 15 Gew.-%, und vorzugsweise weniger als 80 Gew.-%, besonders bevorzugt weniger als 70 Gew.-%.

Der Strom 4, der an der Destillationsvorrichtung K2 in Verfahrensschritt (c) erhalten wird, wird vorzugsweise dampfförmig abgezogen und mit einer Kompressionsvorrichtung V1 und unter Druckerhöhung verdichtet. Dabei wird ein verdichteter Strom 4 erhalten.

Aus dem Strom 4 wird ein Teilstrom 4b flüssig oder gasförmig ausgeschleust, der aus 1- und 2-Butenen neben 1,3-Butadien besteht.

Der verdichtete Hauptstrom 4a, der noch 1,3-Butadien enthält, wird in dem Kondensator W1 kondensiert.

Als Sumpfprodukt erhält man im Verfahrensschritt (c) einen Strom 6, der den mindestens einen Katalysator, den mindestens einen Stabilisator sowie 3-Pentennitrile und 2-Methyl-3-butennitril enthält. Der Anteil an Pentennitril-Isomeren in dem Strom 6 beträgt in Summe vorzugsweise 0,1 bis 80 Gew.-%, besonders bevorzugt 5 bis 50 Gew.-%, insbesondere 10 bis 40 Gew.-%, jeweils bezogen auf den Strom 6.

Darüber hinaus ist es besonders bevorzugt, dass der Strom 6 in den Verfahrensschritt (a) der Hydrocyanierung zumindest teilweise zurückgeführt wird (Strom 6b). Dabei ist es möglich, dass der zurückgeführte Katalysator teilweise einer Regeneration, beispielsweise wie in der deutschen Patentanmeldung DE 10 35 10 02 mit dem Titel, "Einsatz von azeotrop-getrocknetem Nickel(II)-halogenid" der BASF Aktiengesellschaft beschrieben, unterzogen wird.

Der Gehalt an 2-Methyl-3-butennitril in diesem zurückgeführten Strom 6 beträgt in einer bevorzugten Ausführungsform des erfindungsgemäßen Verfahrens weniger als 10 Gew.-%, besonders bevorzugt weniger als 5 Gew.-%, insbesondere weniger als 1 Gew.-%. Dieses wird dadurch erreicht, dass zwischen der Abzugsstelle für Strom 5 und der Abzugsstelle für Strom 6 genügend destillative Trennstufen vorgesehen werden.

Die thermische Belastung des Katalysators kann in einer bevorzugten Ausführungsform dadurch niedrig gehalten werden, dass die Sumpftemperatur 140 °C nicht überschreitet, was durch geeignete Druckverhältnisse sichergestellt werden kann.

Zudem ist es auch möglich, den Strom 6 aus Verfahrensschritt (c) ganz oder teilweise als Katalysatorstrom für andere Hydrocyanierungen zu verwenden, beispielsweise zur Hydrocyanierung von 3-Pentennitril. Auch wenn der Katalysatorstrom 6 zur Hydrocyanierung von 3-Pentennitril verwendet wird, ist es bevorzugt, dass der Gehalt an 2-Methyl-3-butennitril in diesem Katalysatorstrom 6 möglichst gering ist und die zuvor genannten Werte nicht übersteigt.

In einer weiteren bevorzugten Ausführungsform wird ein Frischkatalysatorstrom in die Destillationsvorrichtung von Verfahrensschritt (c) gefahren, um den Pentennitril-Gehalt des gesamten Katalysatorstroms zu Verfahrensschritt (a) in den oben angegebenen Grenzen kontrollieren zu können.

In einer weiteren bevorzugten Ausführungsform des erfindungsgemäßen Verfahrens wird die Menge der Katalysatorausschleusung und damit die nötige Ergänzungsmenge an Frischkatalysator so bemessen, dass im Katalysatorkreislauf der Gehalt an Methlyglutarodinitril nicht über 50 Gew.-%, besonders bevorzugt nicht über 20 Gew.-%, insbesondere nicht über 10 Gew.-%, jeweils bezogen auf den Katalysatorkreislaufstrom, steigt, um den jeweils ausgeschleusten Katalysatorstrom in einer Regenerierung mit möglichst wenig hemmenden Effekten von Methylglutarodinitril zur Aufnahme von Nickel(0) vorliegen zu haben.

In einer weiteren bevorzugten Ausführungsform des erfindungsgemäßen Verfahrens wird die Menge der Katalysatorausschleusung und damit die nötige Ergänzungsmenge an Frischkatalysator so bemessen, dass im Katalysatorkreislauf der Gehalt an Nickel(0)-Komplexen nicht unter 0,05 Gew.-% fällt, besonders bevorzugt nicht unter 0,1 Gew.-%, insbesondere nicht unter 0,2 Gew.-%, jeweils bezogen auf den Katalysatorkreislauf und jeweils berechnet als metallisches Nickel(0), um die Aktivität des Hydrocyanierungskatalysators trotz Verlusten von Nickel(0)-Komplexen während der Reaktion in Schritt (a) oder während der Destillationsverfahren in Schritt (b) und (c), insbesondere während der Reaktion in Schritt (a), sicherzustellen.

In einer weiteren bevorzugten Ausführungsform des erfindungsgemäßen Verfahrens ist es möglich, den Strom 1, der in Verfahrensschritt (a) erhalten wird, unter Ausschluss von Verfahrensschritt (b) direkt in Verfahrensschritt (c) zu überführen.

Nicht ausgenommen vom Umfang dieser Beschreibung sind Verfahrensvarianten, bei denen der Druck der Stufe (e) frei gewählt wird und der Gasstrom 5 gegebenenfalls auf einen höheren Druck als an der Entnahmestelle in (c) verdichtet oder durch Kondensation verflüssigt und gegebenenfalls mit einer Pumpe gefördert wird, um der Stufe (e) zugeführt werden zu können.

Der Verfahrensschritt (e) des erfindungsgemäßen Verfahrens kann in jeder geeigneten, dem Fachmann bekannten Apparatur durchgeführt werden. Für diese Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen oder einstufige Verdampfer, wie Fallfilmverdampfer, Dünnschichtverdampfer, Flashverdampfer, Mehrphasenwendelrohrverdampfer, Naturumlaufverdampfer oder Zwangsumlaufentspannungsverdampfer. Die Destillation kann in mehreren, wie zwei oder drei Apparaturen, vorzugsweise in einer einzigen Apparatur durchgeführt werden.

Die Kolonnen enthalten vorzugsweise strukturierte Packungen. Dabei erzeugen die strukturierten Packungen vorzugsweise 5 bis 100, besonders bevorzugt 10 bis 80, insbesondere 15 bis 50, theoretische Trennstufen.

Der Druck in Verfahrensschritt (e) beträgt vorzugsweise 0,001 bis 100 bar, besonders bevorzugt 0,01 bis 20 bar, insbesondere 0,05 bis 2 bar. Die Destillation wird so durchgeführt, dass die Temperatur im Sumpf der Destillationsvorrichtung vorzugsweise 30 bis 250 °C, besonders bevorzugt 50 bis 200 °C, insbesondere 60 bis 180 °C, beträgt. Die Destillation wird so durchgeführt, dass die Kondensationstemperatur am Kopf der Destillationsvorrichtung vorzugsweise -50 bis 250 °C, besonders bevorzugt 0 bis 180 °C, insbesondere 15 bis 160 °C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten. In einer Ausführungsform des erfindungsgemäßen Verfahrens kann Strom 7, der in dem Verfahrensschritt (e) erhalten wird, einer Isomersierung gemäß der DE-A-102 004 004 671 zugeführt werden.

In einer Ausführungsform des erfindungsgemäßen Verfahrens kann Strom 7, der in dem Verfahrensschritt (e) erhalten wird, in den Verfahrensschritt (a) und/oder in Verfahrensschritt (b) zurückgeführt werden, wobei die Reaktionsbedingungen in Verfahrensschritt (a) oder die Verweilzeit der flüssigen Phase im Sumpf von Verfahrensschritt (b) so gewählt werden, dass 2-Methyl-3-butennitril zumindest teilweise zu trans-3-Pentennitril isomerisiert wird.

In einer weiteren Ausführungsform des erfindungsgemäßen Verfahrens wird der Strom 7 als Seitenabzugsstrom in der Destillationsvorrichtung des Verfahrensschritts (e) gewonnen, wobei als Kopfprodukt dieser Destillationskolonne ein Strom erhalten wird, der neben 2-Methyl-3-butennitril im Wesentlichem noch (Z)-2-Methyl-2-butennitril und gegebenenfalls 1,3-Butadien und Buten-Isomere sowie Vinylcyclohexen und Ethylidencyclohexen enthält. Diese Ausführungsform ist vorteilhaft, da der Strom 7 dann reicher an 2-Methyl-3-butennitril ist als der Kopfstrom.

Der Gehalt an trans-3-Pentennitril in dem Strom 7 beträgt vorzugsweise 0 bis 50 Gew.-%, besonders bevorzugt 100 Gew.-ppm bis 20 Gew.-%, insbesondere 1 bis 15 Gew.-%. Der Gehalt an 2-Methyl-3-butennitril in dem Strom 8 beträgt vorzugsweise 0 bis 10 Gew.-%.

Das erfindungsgemäße Verfahren ermöglicht die Herstellung von 3-Pentennitril und 2-Methyl-3-butennitril in einem integrierten Verfahren, das aufgrund der nahezu vollständig möglichen Rückführung der 1,3-Butadienströme und des Katalysatorstroms eine hohe Verfahrensausbeute für die Einsatzstoffe aufweist. Dabei sind die zur destillativen Abtrennung von 1,3-Butadien und Pentennitril-Isomeren aus den katalysatorhaltigen Strömen nötigen Temperaturen und Druckverhältnisse so wählbar, dass einerseits die Sumpfverdampfertemperaturen bei Ausübung des Verfahrens im Produktionsmaßstab mit technisch erreichbaren Verweilzeiten so niedrig sind, dass sie vorzugsweise nicht zu einer Katalysatorschädigung führen und dass andererseits die Kondensation der Kopfprodukte der jeweiligen Destillationsschritte vorzugsweise bei Temperaturen stattfinden, bei denen die Wärmeabfuhr im Produktionsmaßstab mit wirtschaftlich vertretbarem Aufwand möglich ist.

Neu und erfinderisch ist weiterhin, dass stabilisatorhaltiges Butadien eingesetzt werden kann, das durch Azeotrop-Destillation getrocknet wird. Wenn man zusätzlich eine wäßrige Stabilisator-Lösung (Strom 10) in den oder die Verdichter und/oder Kondensatoren in Stufe (d) einbringt, tritt an keiner Stelle des Verfahrens die Gefahr der Butadien-Polymerisation auf.

Eine Aufpegelung von Stabilisator tritt nicht auf, da dieser, wie in US 3.773.809 beschrieben, über einen Purge-Strom zur Katalysator-Extraktion gefahren wird und dort bei der Phasentrennung mit der Unterphase (ADN-Phase) ausgetragen wird.

Falls der in den Reaktor R1 gefahrene Cyanwasserstoff nicht vollständig umgesetzt wird, besteht keine Gefahr, dass er in die Aufarbeitungsstufen gelangt und dort Feststoffablagerungen, enthaltend Ni(CN)₂, hervorruft. Stattdessen entsteht ein Butadien/Blausäure-Leichtsieder-Azeotrop. Die Blausäure gelangt in den Kopfabzug der Kolonne K 1 und wird über den Kondensator W1 in die Vorrichtung zur Phasentrennung gefahren. Zusammen mit der flüssigen Wasserphase wird sie aus dem Verfahren ausgeschleust.

## Patentansprüche

1. Verfahren zur Herstellung von 3-Pentennitril durch Hydrocyanierung von 1,3-Butadien, **gekennzeichnet durch** die folgenden Verfahrensschritte:
(b') Destillation von Wasser, 1- und 2-Butene und einen Stabilisator enthaltendem 1,3-Butadien in einer Destillationsvorrichtung K4 unter Erhalt eines Stroms 15 als Sumpfprodukt, der getrocknetes 1,3-Butadien, 1- und 2-Butene und den Stabilisator enthält und eines Stroms 16 als Kopfprodukt, der ein azeotropes 1,3-Butadien/Wassergemisch enthält, Kondensation des Stromes 16 in einem Kondensator W, Überführung des erhaltenen Kondensats (Strom 17) in eine Phasentrennvorrichtung, Rückführung der oberen, aus 1,3-Butadien bestehenden flüssigen Phase (Strom 18) als Rücklauf auf die Kolonne K4 und Ausschleusung der unteren flüssigen Wasserphase (Strom 19),
(a) Umsetzung von Strom 15 in einem Reaktor R1 mit Cyanwasserstoff an mindestens einem Katalysator (Strom 6d) unter Erhalt eines Stroms 1, der 3-Pentennitril, 2-Methyl-3-butennitril, den mindestens einen Katalysator in Form homogen gelöster Nickel(0)-Komplexe und phosphorhaltige Liganden, unumgesetztes 1,3-Butadien, 1- und 2-Butene und gegebenenfalls Reste von nicht umgesetztem Cyanwasserstoff enthält,
(b) Destillation des Stromes 1 in einer Destillationsvorrichtung K1 unter Erhalt eines Stromes 2 als Kopfprodukt, der den überwiegenden Teil des 1,3-Butadiens aus Strom 1 enthält, und Erhalt eines Stroms 3 als Sumpfprodukt, der 3-Pentennitril, den mindestens einen Katalysator, 2-Methyl-3-butennitril, 1- und 2-Butene und den restlichen Teil des 1,3-Butadiens aus Strom 1 enthält, der nicht in Strom 2 abgetrennt wurde,
(c) Destillation des Stromes 3 in einer Destillationsvorrichtung K2 unter Erhalt eines Stroms 5 an einem Seitenabzug der Kolonne, der 3-Pentennitril und 2-Methyl-3-butennitril, eines Stroms 6 als Sumpfprodukt, der den mindestens einen Katalysator enthält und eines Stroms 4 als Kopfprodukt,
(d) Verdichtung des Stroms 4 im Verdichter V1, Ausschleusung eines gasförmigen Teilstroms 4b, der 1- und 2-Butene enthält, Überführung des verdichteten Stroms 4a in den Kondensator W1, gemeinsame Kond. Mit Strom 2 aus b) in gemeinsame Kondensation dieses Stromes 2 aus b) und Überführung des Kondensats als Strom 9, teils als Rücklauf auf die Kolonne K1 (Strom 9b), teils als Rückstrom in den Reaktor R1 (Strom 9a) und
(e) Destillative Auftrennung des Stroms 5 unter Erhalt von 3-Pentennitril und 2-Methyl-3-butennitril.

2. Verfahren nach Anspruch 1, **dadurch gekennzeichnet, dass** in Verfahrensschritt (d) eine wässrige Lösung mindestens eines Butadien-Stabilisators in den oder die Verdichter und/oder Kondensatoren eingebracht wird.

3. Verfahren nach den Ansprüchen 1 oder 2, **dadurch gekennzeichnet, dass** aus Strom 6 ein Teilstrom 6a ausgeschleust wird, der den mindestens einen Katalysator und mindestens einen Butadien-Stabilisator enthält.

4. Verfahren nach den Ansprüchen 1 bis 3, **dadurch gekennzeichnet, dass** nicht umgesetzter Cyanwasserstoff mit Strom 12 aus der Phasentrennvorrichtung ausgeschleust wird.

## Claims

1. A process for preparing 3-pentenenitrile by hydrocyanating 1,3-butadiene, which comprises the following process steps:
(b') distillation of 1,3-butadiene comprising water, 1- and 2-butenes and a stabilizer in a distillation apparatus K4 to obtain a stream 15 as the bottom product, which comprises dried 1,3-butadiene, 1- and 2-butenes and the stabilizer, and a stream 16 as the top product, which comprises an azeotropic 1,3-butadiene/water mixture, condensation of the stream 16 in a condenser W, transfer of the resulting condensate (stream 17) into a phase separation apparatus, recycling of the upper liquid phase (stream 18) consisting of 1,3-butadiene as reflux to the column K4, and discharge of the lower liquid aqueous phase (stream 19),
(a) reaction of stream 15 in a reactor R1 with hydrogen cyanide over at least one catalyst (stream 6d) to obtain a stream 1 which comprises 3-pentenenitrile, 2-methyl-3-butenenitrile, the at least one catalyst in the form of homogeneously dissolved nickel(0) complexes and phosphorus ligands, unconverted 1,3-butadiene, and 1- and 2-butenes, with or without residues of unconverted hydrogen cyanide,
(b) distillation of stream 1 in a distillation apparatus K1 to obtain a stream 2 as the top product, which comprises the predominant portion of the 1,3-butadiene from stream 1, and to obtain a stream 3 as the bottom product, which comprises 3-pentenenitrile, the at least one catalyst, 2-methyl-3-butenenitrile, 1- and 2-butenes and the remaining proportion of the 1,3-butadiene from stream 1 which has not been removed in stream 2,
(c) distillation of stream 3 in a distillation apparatus K2 to obtain a stream 5 at a side draw of the column, which 3-pentenenitrile and 2-methyl-3-butenenitrile, a stream 6 as the bottom product, which comprises the at least one catalyst, and a stream 4 as the top product,
(d) compression of stream 4 in the compressor VI, discharge of a gaseous substream 4b which comprises 1- and 2-butenes, transfer of the compressed stream 4a into the condenser W1, combined cond. With stream 2 from b) in combined condensation of this stream 2 from b) and transfer of the condensate as stream 9, partly as reflux to the column K1 (stream 9b), partly as return stream into the reactor R1 (stream 9a), and
(e) distillative separation of stream 5 to obtain 3-pentenenitrile and 2-methyl-3-butenenitrile.

2. The process according to claim 1, wherein, in process step (d), an aqueous solution of at least one butadiene stabilizer is introduced into the compressor(s) and/or condenser(s).

3. The process according to claims 1 or 2, wherein a substream 6a which comprises the at least one catalyst and at least one butadiene stabilizer is discharged from stream 6.

4. The process according to claims 1 to 3, wherein unconverted hydrogen cyanide is discharged from the phase separation apparatus with stream 12.

## Revendications

1. Procédé de fabrication de 3-pentène-nitrile par hydrocyanation de 1,3-butadiène, **caractérisé par** les étapes de procédé suivantes :
(b') la distillation de 1,3-butadiène contenant de l'eau, des 1- et 2-butènes et un stabilisateur dans un dispositif de distillation K4 pour obtenir un courant 15 en tant que produit de fond, qui contient du 1,3-butadiène séché, des 1- et 2-butènes et le stabilisateur, et un courant 16 en tant que produit de tête, qui contient un mélange azéotropique 1,3-butadiène/eau, la condensation du courant 16 dans un condensateur W, le transfert du condensat obtenu (courant 17) dans un dispositif de séparation de phases, le recyclage de la phase liquide supérieure constituée de 1,3-butadiène (courant 18) en tant que reflux dans la colonne K4, et le déchargement de la phase aqueuse liquide inférieure (courant 19),
(a) la mise en réaction du courant 15 dans un réacteur R1 avec du cyanure d'hydrogène sur au moins un catalyseur (courant 6d) pour obtenir un courant 1, qui contient du 3-pentène-nitrile, du 2-méthyl-3-butène-nitrile, le ou les catalyseurs sous la forme de complexes de nickel (0) dissous de manière homogène et de ligands contenant du phosphore, du 1,3-butadiène non réagi, des 1- et 2-butènes, et éventuellement des résidus de cyanure d'hydrogène non réagi,
(b) la distillation du courant 1 dans un dispositif de distillation K1 pour obtenir un courant 2 en tant que produit de tête, qui contient la majeure partie du 1,3-butadiène du courant 1, et pour obtenir un courant 3 en tant que produit de fond, qui contient du 3-pentène-nitrile, le ou les catalyseurs, du 2-méthyl-3-butène-nitrile, des 1- et 2-butènes, et la partie résiduelle du 1,3-butadiène du courant 1 qui n'a pas été séparée dans le courant 2,
(c) la distillation du courant 3 dans un dispositif de distillation K2 pour obtenir un courant 5 au niveau d'un soutirage latéral de la colonne, qui du 3-pentène-nitrile et du 2-méthyl-3-butène-nitrile, un courant 6 en tant que produit de fond, qui contient le ou les catalyseurs, et un courant 4 en tant que produit de tête,
(d) la compression du courant 4 dans le compresseur V1, le déchargement d'un courant partiel gazeux 4b, qui contient des 1- et 2-butènes, le transfert du courant comprimé 4a dans le condensateur W1, la cond. Commune avec le courant 2 de b) dans la condensation commune de ce courant 2 de (b), et le transfert du condensat en tant que courant 9, en partie en tant que reflux dans la colonne K1 (courant 9b), en partie en tant que recyclage dans le réacteur R1 (courant 9a), et
(e) la séparation par distillation du courant 5 pour obtenir du 3-pentène-nitrile et du 2-méthyl-3-butène-nitrile.

2. Procédé selon la revendication 1, **caractérisé en ce qu'**une solution aqueuse d'au moins un stabilisateur de butadiène est introduite dans le ou les compresseurs et/ou condensateurs à l'étape de procédé (d).

3. Procédé selon les revendications 1 ou 2, **caractérisé en ce qu'**un courant partiel 6a qui contient le ou les catalyseurs et au moins un stabilisateur de butadiène est déchargé à partir du courant 6.

4. Procédé selon les revendications 1 à 3, **caractérisé en ce que** le cyanure d'hydrogène non réagi est déchargé avec le courant 12 du dispositif de séparation de phases.
